# EUROPÄISCHE PATENTSCHRIFT

(11) **EP 1 428 498 B1**
(45) Veröffentlichungstag und Bekanntmachung des Hinweises auf die Patenterteilung: **19.12.2007**
(21) Anmeldenummer: 03025584.8
(22) Anmeldetag: 08.11.2003
(51) Int. Cl.: A61K 8/81, A61Q 5/06

(54) **Klare, als Aerosol versprühbare Haarlotion**
Clear sprayable aerosol hair lotion
Lotion claire pour les cheveaux pulvérisable sous forme d'aerosol

(30) Priorität: 11.12.2002 DE 10257858
(43) Veröffentlichungstag der Anmeldung: 16.06.2004
(73) Patentinhaber: Wella Aktiengesellschaft, 64274 Darmstadt (DE)
(72) Erfinder: Schulz, Thomas, 64285 Darmstadt (DE); Wendel, Harald, 64372 Ober-Ramstadt (DE); Birkel, Susanne, Dr., 64285 Darmstadt (DE)

(56) Entgegenhaltungen:
- DE-C1- 19 833 516
- US-A- 4 668 508
- US-A- 6 139 849

## Beschreibung

Gegenstand der Erfindung ist ein Produkt zur Haarbehandlung gemäß Anspruch 1 bestehend aus einer druckfesten Verpackung aus Polyethylenterephtalat oder Glas, einer Vorrichtung zum Versprühen einer in der Verpackung befindlichen Zusammensetzung, einer versprühbaren, flüssigen Zusammensetzung mit einem Gehalt an haarfestigendem Polymer, ausgewählten Alkoholen, 5-50 Gew.% Wasser und ausgewählten Treibmitteln, wobei in der Verpackung neben einer Gasphase nur eine einzige flüssige und klare Phase vorliegt. Das Produkt eignet sich als Hilfsmittel für die Frisurenerstellung.

Sprühprodukte zur Haarbehandlung können unterteilt werden in Haarsprays und Sprühlotionen. Bei Haarsprays, die in der Regel als Aerosole angewendet werden, handelt es sich um Produkte, welche nach einer Frisurengestaltung als Finish-Produkt zur Festigung der erstellten Frisur auf das Haar aufgesprüht werden. Im Gegensatz dazu dienen Lotionen oder Sprühlotionen dazu, die Haare für eine nachfolgende Frisurenerstellung vorzubereiten und werden vor der Frisurengestaltung auf das Haar aufgebracht. Der Wirkmechanismus von Haarsprays und Sprühlotionen ist unterschiedlich. Bei der Anwendung von Haarsprays laufen die einzelnen versprühten Tröpfchen auf den Haaren entlang, sammeln sich an Kreuzungspunkten von Einzelhaaren und verkleben diese nach Verdunstung des Lösungsmittels durch die Abscheidung von festigenden Polymeren. Bei Lotionen spreitet die auf das Haar aufgebrachte Polymerlösung auf dem Haar und bildet nach Verdunstung des Lösungsmittels einen die Einzelhaare oder eine Mehrzahl von Einzelhaaren zumindest teilweise umhüllenden Polymerfilm.
DE 198 33 516 C1 offenbart ein haarfestigendes Mittel, das eine Mischung enthält aus (A) mindestens einem amphoteren Polymer, welches ausgewählt ist aus amphoteren Copolymeren, welche gebildet sind aus mindestens einer ersten Monomerart, welche quaternäre Amingruppen aufweist und mindestens einer zweiten Monomerart, welche Säuregruppen aufweist und (B) mindestens einem Polymeren, welches keine quaternären Amingruppen enthält und gebildet ist aus mindestens einer Monomerart, welche Säuregruppen aufweist.
U.S. Patent Nr. 4,668,508 offenbart eine Zusammensetzung zur Behandlung des Haares. Die Zusammensetzung enthält mindestes ein kationisches Polymer mit einem Molekulargewicht von 500 bis 3 000 000, mindestens ein anionisches Polymer mit einem Molekulargewicht von 500 bis 3 000 000, mindestens einem Zucker und mindestens einem Salz in einem in kosmetischer Hinsicht akzeptablen Medium.

Die der vorliegenden Erfindung zugrunde liegende Aufgabe bestand darin, ein Haarbehandlungsprodukt zur Verfügung zu stellen, welches wie ein Aerosol-Haarspray versprüht wird aber die Anwendungseigenschaften einer Lotion besitzt. Gleichzeitig soll das Produkt in einer kostengünstigen, nicht korrodierbaren und nach Möglichkeit transparenten oder durchsichtigen, vorzugsweise leichten Verpackung auf Kunststoffbasis vorliegen, wobei die Zusammensetzung ausreichend kältestabil ist und in einer attraktiven, einphasigen und klaren Form vorliegt. Die Zusammensetzung benötigt eine Mindestmenge an Wasser zur Sichersellung des notwendigen Spreitverhaltens und Verdunstungsprofils. In der Regel ist eine Kombination von wasserhaltigen Zusammensetzungen mit verflüssigten hydrophoben Treibgasen ungeeignet, um klare Produkte zu bilden, da sich an der Phasengrenze zwischen verflüssigter Treibgasphase und Wirkstoffphase aufgrund von Wechselwirkungen von Treibmittel, Wirk- und Hilfstoffen und Lösungsmitteln ein inhomogener, unkosmetischer und unattraktiv aussehender, Schlieren aufweisender Bereich bildet und/oder es zu Trübungen von einer oder beiden Phasen aufgrund des Zusammenwirkens der üblicherweise eingesetzten Inhaltsstoffe kommt. Wünschenswert sind aber transparente Verpackungen aufgrund der einfachen optischen Erkennbarkeit des Füllstandes sowie allgemein wegen einer größeren Attraktivität von klaren, transparenten Produkten. Ein Ersatz der hydrophoben Treibmittel durch hydrophile Treibmittel wie z.B. Dimethylether ist problematisch aufgrund von Inkompatibilitäten bei Verpackungsmaterialien aus Kunststoffen.

Es wurde nun gefunden, dass die Aufgabe gelöst wird durch ein Produkt zur Haarbehandlung bestehend aus
(A) einer druckfesten Verpackung aus Polyethylenterephtalat oder Glas,
(B) einer Vorrichtung zum Versprühen einer in der Verpackung befindlichen Zusammensetzung,
(C) einer versprühbaren, flüssigen Zusammensetzung mit einem Gehalt an
   (c1) mindestens einem haarfestigenden Polymer gemäß Anspruch 1,
   (c2) mindestens einem einwertigen Alkohol, ausgewählt aus Ethanol, n-Propanol und Isopropanol,
   (c3) 5-50 Gew.% Wasser und
(D) mindestens einem Treibmittel, ausgewählt aus Propan, n-Butan, Isopropan, Fluorkohlenwasserstoffen und Gemischen der genannten Treibmittel,
wobei in der Verpackung neben einer Gasphase nur eine einzige flüssige und klare Phase vorliegt.

Wasser ist in der flüssigen Zusammensetzung (C) des erfindungsgemäßen Produktes beispielsweise in einer Menge von 5 bis 50, insbesondere von 10 bis 40 Gew.% enthalten. Das Mengenverhältnis von flüssiger Zusammensetzung (C) zu Treibmittel (D) beträgt beispielsweise von 96:4 bis 60:40, insbesondere von 93:7 bis 70:30. Die flüssige Phase ist klar im Sinne der Erfindung, wenn mit bloßem Auge keine Trübungen oder Schlieren zu erkennen sind. Eine gute versprühbarkeit liegt insbesondere dann vor, wenn die mittlere Tröpfchengröße, ausgedrückt als dv(50)-Wert, beim Versprühen mittels eines Sprühkopfes DPV Kosmos mit Ring Wirbel .020 und Ventil DPV Kegel 1x .010 Nylon der Firma DPV weniger als 100 µm, vorzugsweise weniger als 90 µm beträgt oder bei der der dv(90) Wert maximal 160 µm, vorzugsweise maximal 150 µm beträgt. Die dv(50)- bzw. dv(90)-Werte geben den maximalen Durchmesser an, den 50% bzw. 90% aller Tröpfchen besitzen. Die Tröpfchengrößenverteilung kann beispielsweise bestimmt werden mit Hilfe eines Partikelmessgerätes auf Basis von Laserstrahlbeugung, z.B. eines Malvern Particle Sizer Messgerätes. Das mit dem erfindungsgemäßen Produkt erzeugbare Spray weist vorzugsweise einen dv(50) Wert im Bereich von 50 bis 100 µm, insbesondere von 70 bis 90 µm und einen dv(90) Wert im Bereich von 90 bis 160 µm, insbesondere von 115 bis 150 µm auf.

Der Gegenstand der vorliegenden Erfindung ist hervorragend dazu geeignet, um Formulierungen mit einem reduzierten Anteil an leicht flüchtigen organischen Bestandteilen (VOC), z.B. VOC 80% oder VOC 55% Formulierungen herzustellen. Das erfindungsgemäße Produkt enthält daher vorzugsweise maximal 80 Gew.%, besonders bevorzugt maximal 55 Gew.% bezogen auf die aus flüssiger Zusammensetzung und Treibmittel bestehende Gesamtzusammensetzung, an Bestandteilen, welche als VOC klassifiziert sind. Das California Air Resources Board (CARB) definiert die VOC als Stoffe mit einem Dampfdruck von > 0,1 mm Hg bei 20°C oder als Stoffe mit 12 oder weniger C-Atomen, wobei eine Reihe von Substanzen bei dieser Definition wegen ihres geringen oder nicht vorhandenen photochemischen Ozonbildungspotentials (Photochemical Ozone Creation Potential, POCP) ausgenommen sind, z.B. Kohlendioxid, Methylenchlorid, Aceton, Methylacetat, FCKWs und fluorierte Kohlenwasserstoffe.

### Haarfestigende Polymere

Das haarfestigende Polymer (c1) ist In der flüssigen Zusammensetzung (C) des erfindungsgemäßen Produktes beispielsweise in einer Menge von 1 bis 15, insbesondere von 2 bis 8 Gew.%, enthalten. Haarfestigende Polymere zeichnen sich dadurch aus, dass sie bei Anwendung in 0,01 bis 5%-iger wässriger, alkoholischer oder wässrig-alkoholischer Lösung in der Lage sind, auf dem Haar einen Polymerfilm abzuscheiden und auf diese Weise das Haar zu festigen.

Die für das erfindungsgemäße Produkt verwendeten haarfestigenden Polymere sind ausgewählt aus Vinylacetat/Crotonsäure Copolymere, welche in nichtionischer, unneutralisierter Form eingesetzt werden. Die Säurezahl liegt z.B. im Bereich von 15-60, insbesondere von 30-40 mg KOH/g. Ein geeignetes Handelsprodukt ist z.B. Aristoflex® A 60.

### Einwertige Alkohole

Der einwertige Alkohol (c2) ist in der flüssigen Zusammensetzung (C) des erfindungsgemäßen Produktes beispielsweise in einer Menge von 45 bis 90 insbesondere von 55 bis 85 Gew.% enthalten. Es können Ethanol, Isopropanol, n-Propanol, Gemische aus Ethanol und Isopropanol, Ethanol und n-Propanol, Isopropanol und n-Propnaol oder Ethanol, Isopropanol und n-Propanol eingesetzt werden. Besonders bevorzugt sind Ethanol und Isopropanol als einzige einwertige Alkohole sowie deren Gemisch. Die flüssige Zusammensetzung kann weitere Lösungsmittel enthalten, sofern die Klarheit der Lösung nicht beeinträchtigt wird. Als weitere einwertige Alkohole könnten z.B. Methanol, Butanole, Pentanole in Frage kommen, oder mehrwertige Alkohole mit 2 bis 6 C-Atomen wie Ethylenglykol, Glycerin, Propylenglykol, Butylenglykol oder Pentandiol.

### Treibmittel

Bei den erfindungsgemäß einzusetzenden Treibmitteln handelt es sich um hydrophobe Stoffe, welche unter Normalbedingungen, d.h. bei Normaldruck (1013 mbar) und Raumtemperatur (20°C) gasförmig sind, bei Abfüllung unter Druck in verflüssigter oder gelöster Form vorliegen. Geeignet sind insbesondere C3-bis C4-Kohlenwasserstoffe wie Propan, n-Butan, Isobutan Fluorkohlenwasserstoffen oder Gemische der genannten Treibmittel. Fluorkohlenwasserstoffe sind z.B. F 152a (1,1-Difluorethan) oder F 134 (Tetrafluorethan). Besonders bevorzugt sind Gemische von Propan und n-Butan, Propan und Isobutan sowie Propan, n-Butan und Isobutan. Im Falle von Kohlenwasserstofftreibmitteln ist der Gehalt an einwertigen Alkoholen in der flüssigen Zusammensetzung vorzugsweise größer oder gleich 70 Gew.% und der Gehalt an Wasser vorzugsweise kleiner oder gleich 25 Gew.%. Im Falle von Fluorkohlenwasserstofftreibmitteln ist der Gehalt an einwertigen Alkoholen in der flüssigen Zusammensetzung vorzugsweise größer oder gleich 60 Gew.% und der Gehalt an Wasser vorzugsweise kleiner oder gleich 35 Gew.%. Art und Menge der Treibmittel sind vorzugsweise so gewählt, dass in der Verpackung bei 20°C ein Druck von z.B. 1 bis 3, insbesondere von 2 bis 2,7 bar herrscht.

### Verpackung

Das erfindungsgemäße Produkt ist in einer geeigneten, druckdichten Aerosolverpackung verpackt und weist als zusätzliche Komponente eine Vorrichtung auf, welche das Versprühen der Zusammensetzung unter Verwendung des Treibmittels ermöglicht. Als geeignete Sprühvorrichtung kann beispielsweise ein handelsüblicher Aerosolsprühkopf verwendet werden. Die Verpackung ist aus Polyethylenterephtalat und vorzugsweise transparent oder durchsichtlg, sodass die Konsistenz, die Füllstandshöhe, die Farbe der Zusammensetzung der flüssigen Phase von außen erkennbar ist. Ein anderes, transparentes oder durchsichtiges Material ist z.B. Glas.

### Weitere Zusatzstoffe

Das erfindungsgemäße Mittel kann darüber hinaus die für Haarbehandlungsmittel üblichen Zusatzbestandteile enthalten, soweit die Klarheit des Produktes nicht beeinträchtigt wird, z.B. Konservierungsmittel, Emulgatoren, Lösungsvermittler, Parfümöle, Duftstoffe, pH-Puffersubstanzen, Pfiegestoffe wie z.B. Pflanzen- und Kräuterextrakte, Protein- und Seidenhydrolysate, Silikonverbindungen, Lichtschutzmittel, Antioxidantien, Radikalfänger, Antischuppenwirkstoffe, Glanzgeber, Vitamine, Weichmacher, Kämmbarkeitsverbesserer, Anfärbemittel etc..

Eine besonders bevorzugte Ausführungsform ist ein Produkt, bei welchem in der flüssigen Zusammensetzung
(c1) 1 bis 8 Gew.% eines nicht neutralisierten Vinylacetat/Crotonsäure Copolymers
(c2) 70 bis kleiner 90 Gew.% mindestens eines einwertigen Alkohols, ausgewählt aus Ethanol, n-Propanol und Isopropanol und
(c3) größer 10 bis 25 Gew.% Wasser enthalten sind,
das Treibmittel ausgewählt ist aus Propan, n-Butan, Isobutan und deren Gemischen, das Mengenverhältnis von flüssiger Zusammensetzung zu Treibmittel von 93:7 bis 70:30 beträgt und der Druck in der Verpackung maximal 3 bar bei 20°C beträgt.

Eine weitere bevorzugte Ausführungsform ist ein Produkt, bei welchem in der flüssigen Zusammensetzung
(c1) 1 bis 8 Gew.% eines nicht neutralisierten Vinylacetat/Crotonsäure Copolymers
(c2) 60 bis kleiner 90 Gew.% mindestens eines einwertigen Alkohols, ausgewählt aus Ethanol, n-Propanol und Isopropanol und
(c3) 10 bis 35 Gew.% Wasser enthalten sind,
das Treibmittel ausgewählt ist aus fluorierten Kohlenwasserstoffen, das Mengenverhältnis von flüssiger Zusammensetzung zu Treibmittel von 93:7 bis 70:30 beträgt und der Druck in der Verpackung maximal 3 bar bei 20 °C beträgt.

Gegenstand der Erfindung ist auch ein Verfahren zur Haarbehandlung, wobei eine Zusammensetzung mittels eines erfindungsgemäßen Produkts auf das Haar aufgebracht wird und anschließend eine Frisurenerstellung erfolgt.

Das erfindungsgemäße kosmetische Produkt wird angewendet, indem eine in Abhängigkeit von der Haarmenge und des Haarzustands zur Erzielung des gewünschten haarfestigenden Effektes ausreichende Menge der Zusammensetzung (typischerweise ca. 1 - 10 g, insbesondere 2 bis 7 g) auf das Haar aufgesprüht wird. Die gewünschte Frisur wird erstellt und das Haar wird gegebenenfalls getrocknet. Abschließend kann ein herkömmliches Haarspray als Finish zur Fixierung der erstellten Frisur angewendet werden.

Das Produkt kann auf feuchtem und auf trockenem Haar angewendet werden. Es kann als Fönlotion und zum Einlegen im Friseursalon oder zu Hause verwendet werden. Das Produkt kann auch zum Auffrischen der Frisur dienen oder auf das trockene, bereits eingelegte Haar aufgesprüht und dann getrocknet werden.

Die nachfolgenden Beispiele sollen den Gegenstand der Erfindung näher erläutern.

### Beispiele

### Beispiel 1: Aerosol-Lotion

| | |
|---|---|
| 5 g | Aristoflex^{®} A 60 ¹⁾ |
| 80 g | Ethanol |
| 0,2 g | Parfüm |
| ad 100 g | Wasser |

| | |
|---|---|
| ¹⁾ 60%ige Lösung von Vinylacetat/Crotonsäure Copolymer in 35% Isopropanol und 5 % Wasser | |

Die flüssige Wirkstofflösung wird mit einem Propan/Butan - Gemisch (2,7 bar) im Verhältnis 90:10 in einer druckfesten Verpackung aus transparentem Polyethylenterephtalat oder aus Glas abgefüllt und mit einem Aerosolsprühkopf versehen.

### Beispiel 2: Aerosol-Lotion (80% VOC)

| | |
|---|---|
| 5,6 g | Aristoflex^{®} A 60 ¹⁾ |
| 75,6 g | Ethanol |
| 0,2 g | Parfüm |
| ad 100 g | Wasser |

Die flüssige Wirkstofflösung wird mit einem Propan/Butan - Gemisch (2,7 bar) im Verhältnis 90:10 in einer druckfesten Verpackung aus transparentem Polyethylenterephtalat abgefüllt und mit einem Aerosolsprühkopf versehen.

### Beispiel 3: Aerosol-Lotion (55% VOC)

| | |
|---|---|
| 6 g | Aristoflex^{®} A 60 ¹⁾ |
| 66 g | Ethanol |
| 0,2 g | Parfüm |
| ad 100 g | Wasser |

Die flüssige Wirkstofflösung wird mit dem Fluorkohlenwasserstoff 152A (1,1-Difluorethan) im Verhältnis 80:20 in einer druckfesten Verpackung aus transparentem Polyethylenterephtalat abgefüllt und mit einem Aerosolsprühkopf versehen.

## Patentansprüche

1. Produkt zur Haarbehandlung bestehend aus
(A) einer druckfesten Verpackung aus Polyethylenterephtalat oder aus Glas,
(B) einer Vorrichtung zum Versprühen einer in der Verpackung befindlichen Zusammensetzung,
(C) einer versprühbaren, flüssigen Zusammensetzung mit einem Gehalt an
(c1) mindestens einem haarfestigenden Polymer, wobei das haarfestigende Polymer ein nicht neutralisiertes Vinylacetat/Crotonsäure Copolymer ist.
(c2) mindestens einem einwertigen Alkohol, ausgewählt aus Ethanol, n-Propanol und Isopropanol,
(c3) 5-50 Gew.% Wasser und
(D) mindestens einem Treibmittel, ausgewählt aus Propan, n-Butan, Isobutan und Fluorkohlenwasserstoffen,
wobei in der Verpackung neben einer Gasphase nur eine einzige flüssige und klare Phase vorliegt.

2. Produkt nach Anspruch 1, **dadurch gekennzeichnet, dass** das haarfestigende Polymer in der Zusammensetzung in einer Menge von 1 bis 15 Gew.% enthalten ist.

3. Produkt nach einem der vorhergehenden Ansprüche, **dadurch gekennzeichnet, dass** der einwertige Alkohol in der Zusammensetzung in einer Menge von 45 bis 90 Gew.% enthalten ist.

4. Produkt nach einem der vorhergehenden Ansprüche, **dadurch gekennzeichnet, dass** das Mengenverhältnis von flüssiger Zusammensetzung zu Treibmittel von 96:4 bis 60:40 beträgt.

5. Produkt nach einem der vorhergehenden Ansprüche, **dadurch gekennzeichnet, dass** der Druck in der Verpakkung maximal 3 bar bei 20 °C beträgt.

6. Produkt nach Anspruch 1, **dadurch gekennzeichnet, dass** in der flüssigen Zusammensetzung
(c1) 1 bis 8 Gew.% eines nicht neutralisierten Vinylacetat/Crotonsäure Copolymers
(c2) 70 bis kleiner 90 Gew.% mindestens eines einwertigen Alkohols, ausgewählt aus Ethanol, n-Propanol und Isopropanol und
(c3) 10 bis 25 Gew.% Wasser enthalten ist,
das Treibmittel ausgewählt ist aus Propan, n-Butan, Isobutan und deren Gemischen, das Mengenverhältnis von flüssiger Zusammensetzung zu Treibmittel von 93:7 bis 70:30 beträgt und der Druck in der Verpackung maximal 3 bar bei 20 °C beträgt.

7. Produkt nach Anspruch 1, **dadurch gekennzeichnet, dass** in der flüssigen Zusammensetzung
(c1) 1 bis 8 Gew.% eines nicht neutralisierten Vinylacetat/Crotonsäure Copolymers
(c2) 60 bis kleiner 90 Gew.% mindestens eines einwertigen Alkohols, ausgewählt aus Ethanol, n-Propanol und Isopropanol und
(c3) 10 bis 35 Gew.% Wasser enthalten ist, das Treibmittel ausgewählt ist aus fluorierten Kohlenwasserstoffen, das Mengenverhältnis von flüssiger Zusammensetzung zu Treibmittel von 93:7 bis 70:30 beträgt und der Druck in der Verpackung maximal 3 bar bei 20 °C beträgt.

8. Verfahren zur Haarbehandlung, wobei eine Zusammensetzung mittels eines Produkts gemäß einem der Ansprüche 1 bis 7 auf das Haar aufgebracht wird und anschließend eine Frisurenerstellüng erfolgt.

## Claims

1. Hair-treatment product consisting of
(A) a pressure-resistant packaging made of polyethylene terephthalate or glass,
(B) a device for spraying a composition located in the packaging,
(C) a sprayable, liquid composition with a content of
(c1) at least one hair-setting polymer, where the hair-setting polymer is an unneutralized vinyl acetate/crotonic acid copolymer,
(c2) at least one monohydric alcohol, selected from ethanol, n-propanol and isopropanol,
(c3) 5-50% by weight of water and
(D) at least one propellent, selected from propane, n-butane, isobutane and fluorohydrocarbons,
where, in the packaging, besides a gas phase, only a single liquid and clear phase is present.

2. Product according to Claim 1, **characterized in that** the hair-setting polymer is present in the composition in an amount of from 1 to 15% by weight.

3. Product according to one of the preceding claims, **characterized in that** the monohydric alcohol is present in the composition in an amount of from 45 to 90% by weight.

4. Product according to one of the preceding claims, **characterized in that** the quantitative ratio of liquid composition to propellent is from 96:4 to 60:40.

5. Product according to one of the preceding claims, **characterized in that** the pressure in the packaging is at most 3 bar at 20°C.

6. Product according to Claim 1, **characterized in that**, in the liquid composition,
(c1) 1 to 8% by weight of an unneutralized vinyl acetate/crotonic acid copolymer
(c2) 70 to less than 90% by weight of at least one monohydric alcohol, selected from ethanol, n-propanol and isopropanol and
(c3) 10 to 25% by weight of water is present,
the propellent is selected from propane, n-butane, isobutane and mixtures thereof, the quantitative ratio of liquid composition to propellent is from 93:7 to 70:30 and the pressure in the packaging is at most 3 bar at 20°C.

7. Product according to Claim 1, **characterized in that**, in the liquid composition,
(c1) 1 to 8% by weight of an unneutralized vinyl acetate/crotonic acid copolymer
(c2) 60 to less than 90% by weight of at least one monohydric alcohol, selected from ethanol, n-propanol and isopropanol and
(c3) 10 to 35% by weight of water is present,
the propellent is selected from fluorinated hydrocarbons, the quantitative ratio of liquid composition to propellent is from 93:7 to 70:30 and the pressure in the packaging is at most 3 bar at 20°C.

8. Method of treating hair, where a composition is applied to the hair by means of a product according to one of Claims 1 to 7 and then a hairstyle is created.

## Revendications

1. Produit pour le traitement des cheveux, constitué de
(A) un emballage tenant la pression, en poly-(éthylène-téréphtalate) ou en verre,
(B) un dispositif pour la pulvérisation d'une composition se trouvant dans l'emballage,
(C) une composition liquide pulvérisable ayant une teneur en
(c1) au moins un polymère fixant les cheveux, le polymère fixant les cheveux étant un copolymère acétate de vinyle/acide crotonique non neutralisé
(c2) au moins un alcool monohydrique choisi parmi l'éthanol, le n-propanol et l'isopropanol,
(c3) 5-50 % en poids d'eau et
(D) au moins un propulseur choisi parmi le propane, le n-butane, l'isobutane et des hydrocarbures fluorés,
outre une phase gazeuse, une unique phase liquide et limpide étant seulement présente dans l'emballage.

2. Produit selon la revendication 1, **caractérisé en ce que** le polymère fixant les cheveux est contenu en une quantité de 1 à 15 % en poids dans la composition.

3. Produit selon l'une quelconque des revendications précédentes, **caractérisé en ce que** l'alcool monohydrique est contenu en une quantité de 45 à 90 % en poids dans la composition.

4. Produit selon l'une quelconque des revendications précédentes, **caractérisé en ce que** le rapport des quantités de la composition liquide au propulseur va de 96:4 à 60:40.

5. Produit selon l'une quelconque des revendications précédentes, **caractérisé en ce que** la pression dans l'emballage est au maximum de 3 bars à 20 °C.

6. Produit selon la revendication 1, **caractérisé en ce que** la composition liquide contient
(c1) 1 à 8 % en poids d'un copolymère acétate de vinyle/acide crotonique non neutralisé
(c2) 70 à moins de 90 % en poids d'au moins un alcool monohydrique choisi parmi l'éthanol, le n-propanol et l'isopropanol,
(c3) 10 à 25 % en poids d'eau,
le propulseur est choisi parmi le propane, le n-butane, l'isobutane et des mélanges de ceux-ci, le rapport des quantités de la composition liquide au propulseur va de 93:7 à 70:30 et la pression dans l'emballage est au maximum de 3 bars à 20 °C.

7. Produit selon la revendication 1, **caractérisé en ce que** la composition liquide contient
(c1) 1 à 8 % en poids d'un copolymère acétate de vinyle/acide crotonique non neutralisé
(c2) 60 à moins de 90 % en poids d'au moins un alcool monohydrique choisi parmi l'éthanol, le n-propanol et l'isopropanol, et
(c3) 10 à 35 % en poids d'eau, le propulseur est choisi parmi des hydrocarbures fluorés, le rapport des quantités de la composition liquide au propulseur va de 93:7 à 70:30 et la pression dans l'emballage est au maximum de 3 bars à 20 °C.

8. Procédé pour le traitement des cheveux, dans lequel on applique sur les cheveux une composition au moyen d'un produit selon l'une quelconque des revendications 1 à 7 et on effectue ensuite l'élaboration d'une coiffure.
